# EUROPEAN PATENT SPECIFICATION

(11) **EP 4 054 519 B1**
(45) Date of publication and mention of the grant of the patent: **09.08.2023**
(21) Application number: 20803108.8
(22) Date of filing: 02.11.2020
(51) Int. Cl.: A61K 8/49, A61Q 11/00, A61K 8/60, A61K 8/81

(54) **ORAL CARE COMPOSITION FOR WHITENING TEETH**
MUNDPFLEGEZUSAMMENSETZUNG ZUR ZAHNAUFHELLUNG
COMPOSITION D'HYGIÈNE BUCCALE POUR LE BLANCHIMENT DES DENTS

(30) Priority: 06.11.2019 WO PCT/CN2019/115945; 09.12.2019 EP 19214518
(43) Date of publication of application: 14.09.2022
(73) Proprietor: Unilever IP Holdings B.V., 3013 AL Rotterdam (NL); Unilever Global IP Limited, Wirral, Merseyside CH62 4ZD (GB)
(72) Inventor: JIN, Huajin, Shanghai 201102 (CN); LI, Yajuan, Shanghai (CN)
(74) Representative: Tansley, Sally Elizabeth
(86) International application number: PCT/EP2020/080615
(87) International publication number: WO 2021/089445

(56) References cited:
- EP-A1- 1 935 395
- WO-A1-2017/105381
- US-A- 5 690 911

## Description

### Field of the Invention

The present invention is concerned with oral care compositions comprising polymers, surfactants and pigments. In particular, the invention is also concerned with the use of such compositions for whitening teeth of an individual.

### Background of the Invention

The colour of teeth is influenced by a combination of their intrinsic colour and the presence of any extrinsic stains that may form on the tooth surfaces. Many substances we consume daily can cause extrinsic stain formation including certain foods, tobacco products, and fluids such as tea and coffee. These discolouring substances are often able to permeate the enamel layer, which ultimately cause staining.

Tooth whiteness is of great interest to consumers, driven by the dissatisfaction with their current tooth colour and desire for whiter teeth. This desire for whiter teeth has given rise to a growing trend in the increased use of tooth whitening products which range from toothpastes to mouthwashes and chewing gums.

Tooth whitening products on the market generally improve tooth colour by essentially two types of mechanisms, namely tooth bleaching or the removal and control of extrinsic stain. Tooth bleaching typically involves the application of hydrogen peroxide or carbamide peroxide containing gels to the teeth using paint-on, mouth guard or strip product formats. The removal and control of extrinsic stain is possible via toothpaste and in particular tooth whitening formulations, which typically contain optimized abrasive and chemical ingredients to maximise stain removal and prevention. Many types of ingredients have been described in the literature for removing and preventing extrinsic stain and these include abrasives, surfactants, calcium chelators, enzymes and polymers, of which abrasive is the primary stain removal ingredient in toothpaste. But it can cause damage to teeth or gums if overused so is often not desired.

It has been known in the literature that the whitening effect can also be produced by changing the visual perception of the color of the teeth. For example, the visual perception of a white substance can be altered through the deposition of an optical brightener, blue pigment or blue dye. Blue Covarine is a phthalocyanine blue pigment present in the toothpaste named white Now^{®} and there have been attempts to enhance the deposition of this pigment on teeth for instant whitening benefits in oral care products.

WO 2016/099544 A1 (Colgate-Palmolive Company) discloses a tooth whitening oral care composition made by combining ingredients comprising flakes of a water soluble whitening film, a dye with a hue angle in the CIELAB system ranging from 200 to 320 degrees and an orally acceptable carrier vehicle.

EP 1 935 395 A1 (Unilever) discloses from 0.01 to 0.3% by weight of a pigment having a hue angle, h, in the CIELAB system of from 220 to 320 degrees, characterized in that the composition further comprises a soluble deposition aid for said pigment.

WO 2012/123241 A2 (Unilever) discloses an oral care composition suitable for delivering a temporary whitening effect to the surface of teeth, the composition comprising: a continuous phase comprising water or polyhydric alcohol or a mixture thereof; a particulate tooth surface whitening agent which is dispersed in the continuous phase, and a deposition aid for the particulate tooth surfaces whitening agent; characterised in that the deposition aid is a poly-(sulphonic acid) polymer. The particulate tooth whitening agent is a phthalocyanine blue pigment.

The present inventors have now found surprisingly that a combination of certain polymers, surfactants and pigments can enhance the instant tooth whitening effect of an oral care composition.

### Summary of the Invention

In a first aspect, the present invention is directed to an oral care composition comprising:
(a) a copolymer;
(b) an anionic surfactant;
(c) an alkyl glucoside; and
(d) a pigment having a hue angle, h, in the CIELAB system of from 220 to 320 degrees; wherein the copolymer comprises:
   (i) a carboxylic monomer A represented by the formula (1):

      CH₂=C(R¹)-COOH (1)

      wherein R¹ is a substituent selected from the group consisting of hydrogen, halogen, hydroxyl, lactone, lactam, the cyanogen groups, monovalent alkyl radical, monovalent alkaryl radicals and monovalent cycloaliphatic radicals;
   (ii) an acrylic ester monomer B represented by the formula (2):

      CH₂=C(R²)-CO(O)-R³ (2)

wherein R² is hydrogen, methyl group or ethyl group, R³ is an alkyl group having from 10 to 30 carbon atoms, preferably from 12 to 22 carbon atoms; and
wherein the anionic surfactant and the alkyl glucoside are present in a weight ratio ranging from 1:8 to 8:1.

In a second aspect, the present invention is directed to a packaged oral care product comprising the oral care composition of the first aspect of this invention.

In a third aspect, the present invention is directed to a method of whitening teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

The copolymer suitable for use in this invention comprises constituting units of two monomers A and B. Monomer A is a carboxylic monomer having a general structure represented by the formula (1)

CH₂=C(R¹)-COOH (1)

wherein R¹ is a substituent selected from the group consisting of hydrogen, halogen, hydroxyl, lactone, lactam, the cyanogen groups, monovalent alkyl radical, monovalent alkaryl radicals and monovalent cycloaliphatic radicals. Preferred monomer A is acrylic acid, methacrylic acid or ethacrylic acid, especially preferred is acrylic acid.

Monomer B is an acrylic ester monomer having a general structure represented by the formula (2):

CH₂=C(R²)-CO(O)-R³ (2)

wherein R² is hydrogen, methyl group or ethyl group, R³ is an alkyl group having from 10 to 30 carbon atoms, preferably from 12 to 22 carbon atoms. Representative monomer B is decyl acrylate, lauryl acrylate, stearyl acrylate, behenyl acrylate, myristyl acrylate or the corresponding methacrylates.

The amount of monomer A is typically from 80 to 99%, more preferably from 90 to 98% by weight of the total monomer content of the copolymer. The amount of monomer B is preferably from 1 to 20%, more preferably from 2 to 10% by weight of the total monomer content of the copolymer.

The copolymer is crosslinked with allyl pentarythritol or allyl sucrose, preferably ally pentaerythritol. The INCI name of the copolymer is acrylates/C₁₀-C₃₀ alkyl acrylate crosspolymer and are commercially available as Carbopol^{®} 1342 NF, Pemulen^{™} TR-1 NF, Pemulen^{™} TR-2 NF, Carbopol^{®} Ultrez 21 from Lubrizol Corporation.

The copolymer, when tested in the form of 0.2% emulsion, has a viscosity of from 500 to 50,000 centipoise, preferably from 800 to 40,000 centipoise and more preferably from 1,000 to 35,000 centipoise. When tested in the form of 1% aqueous mucilage, the copolymer has a viscosity of from 1,000 to 100,000 centipoise, preferably from 2,000 to 80,000 centipoise and more preferably from 3,000 to 50,000 centipoise. The viscosity is measured using the Brookfield RVT Model Viscometer at spindle speed of 20 rpm in the pH range of 7.3 to 7.8 at 25°C.

The composition typically comprises from 0.01 to 10% by weight of the copolymer, more preferably from 0.02 to 5% and most preferably from 0.05 to 3%, based on total weight of the oral care composition and including all ranges subsumed therein.

The anionic surfactant suitable for use in the present application includes, for example, sodium, potassium, ammonium, substituted ammonium salts of C₈-C₁₈ alkyl sulfates (for example sodium lauryl sulfate) or C₈-C₁₈ ethoxylated alkyl sulfates (for example sodium lauryl ether sulfate), C₈ to C₁₈ alkyl sulphosuccinates (for example dioctyl sodium sulphosuccinate), C₈ to C₁₈ alkyl sulphoacetates (for example sodium lauryl sulphoacetate), C₈ to C₁₈ alkyl sarcosinates (for example sodium lauryl sarcosinate), C₈ to C₁₈ alkyl phosphates (which can optionally comprise up to 10 ethylene oxide and/or propylene oxide units) and sulphated monoglycerides, preferably the anionic surfactant is sodium, potassium, ammonium, substituted ammonium salts of alkyl sulfates having 8 to 18 carbon atoms, more preferably 12 to 18 carbon atoms, particularly preferred anionic surfactant is sodium lauryl sulfate.

The anionic surfactant is preferably present at a level of from 0.01 to 15% by weight of the composition, more preferably from 0.1% to 10%, most preferably from 0.5% to 7%, based on total weight of the oral care composition and including all ranges subsumed therein.

Alkyl glucosides are non-ionic surfactants formed by reacting an alcohol or mixtures of alcohols with a cyclic form of the sugar, glucose or glucose polymers. The chemical structure of the alkyl glucoside suitable for use in the compositions of the present invention is represented by the general formula (3)

R⁴-O-(G)ₙ-H (3)

wherein R⁴ is a linear or branched alkyl group comprising from 6 to 22 carbon atoms, preferably from 8 to 14, more preferably from 8 to 12. G is an anhydroglucose unit and the degree of polymerisation, n, is a number from 1 to 10 or more, preferably from 1 to 2.5, more preferably from 1 to 1.5, and most preferably 1. It should be noted that the carbon chain length will typically exhibit a normal chain length distribution. For example, a C₁₂ alkyl glucoside will typically contain residues of other carbon chain lengths unless the product has been completely purified.

Suitable alkyl glucosides that may be used in this invention include, but not limited to, hexyl glucoside, octyl glucoside, decyl glucoside, lauryl glucoside, isodecyl glucoside, coco glucoside and the corresponding oligosaccharides, mixtures thereof or the like. The alkyl glucoside preferably comprises decyl glucoside, lauryl glucoside, coco glucoside and mixtures thereof. Lauryl glucoside is especially preferred.

The alkyl glucoside is typically present at a level of from 0.01 to 15% by weight of the composition, more preferably from 0.1% to 10%, most preferably from 0.5% to 7%, based on total weight of the oral care composition and including all ranges subsumed therein.

The weight ratio between the anionic surfactant and the alkyl glucoside ranges from 1:8 to 8:1, preferably from 1:5 to 5:1, more preferably from 1:3 to 3:1.

The composition also comprises a pigment. The pigment according to the invention is a shade/material which is insoluble in the relevant medium, at the relevant temperature. This is in contrast to dyes which are soluble. The term "soluble", as used herein, means a source that dissolves in water to give a solution with a concentration of at least 0.1 moles per litre. The term "relevant medium", as used herein, refers to human saliva, the liquid medium in which the composition is used, at the temperature of the oral cavity during brushing of the teeth, i.e. up to 37°C. The relevant medium may also be water and the relevant temperature to be 25°C. The only limitation with respect to the pigment is that the same is suitable for use in the mouth.

The pigment has a hue angle, h, in the CIELAB system of from 220 to 320 degrees, more preferably from 250 to 290 degrees. A detailed description of hue angles may be found on p57 of Colour Chemistry 3rd edition by H. Zollinger published by Wiley-VCH. Preferably the pigment is violet or blue, more preferably the pigment is selected from one or more of those listed in the Colour Index International as pigment blue 1 through to pigment 83 and pigment violet 1 through to pigment violet 56. Other suitable pigments are pigment ultramarine blue and ultramarine violet. While the preferred pigment is blue or violet, the same effect may be achieved through mixing pigments outside of this hue angle range. For example, such a hue angle may also be obtained by mixing a red and green-blue pigment to yield a blue or violet shaded pigment.

It is particularly preferred that the pigment is a blue pigment. Some examples of blue pigments suitable for use in this invention include inorganic blue pigments such as iron blue (C.I. 77510) and ultramarine blue (C.I. 77007). A preferred class of blue pigments suitable for use in the invention are organic blue pigments such as phthalocyanine blue pigments. Phthalocyanines are organometallic compounds containing four symmetrically arranged isoindole rings connected in a 16-membered ring linking with alternate carbon and nitrogen atoms. Most phthalocyanines contains a central, coordinated metal ion such as copper. Copper phthalocyanines have the basis structure:

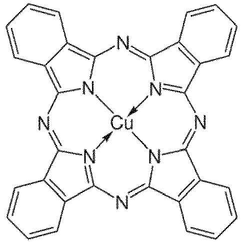

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule. The following pigments are illustrative phthalocyanine blue pigments preferably included in the composition according to the invention:

| **C.I. Generic Name** | **C.I. Constitution Number** | **Crystal modification; Type** | **Number of halogen atoms** |
|---|---|---|---|
| Pigment Blue 15 | 74160 | alpha | -- |
| Pigment Blue 15:1 | 74160 | alpha; phase stabilised | 0.5-1 Cl |
| Pigment Blue 15:2 | 74160 | alpha; phase & flocculation stabilised | 0.5-1 Cl |
| Pigment Blue 15:3 | 74160 | beta | -- |
| Pigment Blue 15:4 | 74160 | beta; flocculation stabilised | -- |
| Pigment Blue 15:6 | 74160 | epsilon | -- |
| Pigment Blue 16 | 74100 | gamma; metal-free | -- |

It is especially preferred that the pigment is phthalocyanine blue pigment selected from alpha copper phthalocyanines Pigment Blue 15, Pigment Blue 15:1, Pigment 15:2 and mixtures thereof. Most preferably the pigment is Pigment Blue 15:1. A commercially available example is Cosmenyl Blue A4R from Clariant.

The pigment suitable for use in this invention may also be mixtures of any of the above described materials.

The oral care composition of the present invention typically comprises from 0.001 to 1% by weight of the pigment, more preferably from 0.01 to 0.5%, and most preferably from 0.02 to 0.2%, based on total weight of the oral care composition and including all ranges subsumed therein.

The oral care composition of the invention may comprise other polymeric materials in addition to the copolymer included in the composition. Particularly preferred polymeric materials include copolymers of methyl vinyl ether and maleic anhydride, high molecular weight PEGs, high molecular weight cellulose ethers or mixtures thereof. "High molecular weight", as used herein, means the polymeric material has a molecular weight of at least 100,000 g/mol, preferably at least 200,000 g/mol. Copolymers of methyl vinyl ether and maleic anhydride are especially preferred, which are commercially available under the trade name GANTREZ^{™}.

Preferably, the oral care composition has a pH from 4.0 to 10.0, more preferably from 5.0 to 9.0, and most preferably from 5.5 to 8.0. The pH of oral are composition is measured when 5 parts by weight of the composition is uniformly dispersed and/or dissolved in 20 parts by weight pure water at 25°C. In particular, the pH may be measured by manually mixing 5 g oral care composition with 20 mL water for 30 s, then immediately testing the pH with indicator or a pH meter.

The oral care composition of the present invention may comprise a physiologically acceptable carrier. Water is the most common carrier for this invention. The carrier may further comprise at least thickener, humectant or a combination thereof.

Thickener may be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, starch, modified starch, silica based thickeners including silica aerogels, magnesium aluminum silicate (e.g., Veegum) and mixtures thereof.

Xanthan gum and/or sodium carboxymethyl cellulose is/are typically preferred. The copolymer which is included in the composition can also behave as a thickener.

In an especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The carrier is typically present at a level of from 10 to 90% by weight of the oral care composition, more preferably 25 to 80%, and most preferably from 30 to 70% by weight of the composition, based on total weight of the composition and including all ranges subsumed therein.

The oral care composition of the present invention may contain a variety of other ingredients which are common in the art to enhance physical properties and performance, in addition to pigments which are included in the composition. These ingredients include antimicrobial agents, anti-inflammatory agents, anti-caries agents, plaque buffers, fluoride sources, dyes, vitamins, plant extracts, desensitizing agents, anti-calculus agents, biomolecules, flavors, proteinaceous materials, preservatives, opacifying agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of whitening the teeth of an individual comprising applying the composition to at least one surface of the teeth of the individual. The method is non-therapeutic. The oral care composition of this invention may additionally or alternatively be for used for whitening the teeth of an individual, and/or used in the manufacture of a medicament in whitening teeth of an individual. The use is non-therapeutic.

Typically, the composition will be packaged. In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The oral care composition of the invention is preferably a toothpastes or gel. When the oral care composition is a toothpaste or gel, the same typically has a viscosity from about 30,000 to 180,000 centipoise, and preferably, from 60,000 to 170,000 centipoise, and most preferably, from 65,000 to 165,000 centipoise, taken at room temperature (25°C) with a Brookfield Viscometer, Spindle No.93/94 and at a speed of 5 rpm for 1 minute. The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day.

The following examples are provided to facilitate an understanding of the present invention.

### Examples

### Example 1

This example demonstrated the effect of polymers on tooth whitening efficacy. All ingredients are expressed by weight percent of the total formulation and as level of active ingredient.

**Table 1**

| **Ingredient** | **Samples** | | | |
|---|---|---|---|---|
| | **1** | **2** | **3** | **4** |
| Water | Balance | Balance | Balance | Balance |
| Sorbitol 70/70 | 45.00 | 45.00 | 45.00 | 45.00 |
| PEG-32 | 2.00 | 2.00 | 2.00 | 2.00 |
| Sodium saccharin | 0.20 | 0.20 | 0.20 | 0.20 |
| Trisodium phosphate | 0.025 | 0.025 | 0.025 | 0.025 |
| Blue Covarine^{a} | 0.025 | 0.025 | 0.025 | 0.025 |
| Pemulen^{™} TR-1 | 0.10 | 0.10 | -- | -- |
| Carbopol^{®} 971P NF | -- | -- | 0.10 | 0.10 |
| Thickening Silica | 8.2375 | 8.2375 | 8.2375 | 8.2375 |
| Abrasive Silica | 8.00 | 8.00 | 8.00 | 8.00 |
| Sorbosil AC 33 | 2.00 | 2.00 | 2.00 | 2.00 |
| Sodium lauryl sulfate | 1.70 | 0.85 | 1.70 | 0.85 |
| Lauryl glucoside^{b} | -- | 0.85 | -- | 0.85 |
| Perfume | 1.10 | 1.10 | 1.10 | 1.10 |
| Sodium carboxymethyl cellulose | 0.795 | 0.795 | 0.795 | 0.795 |

| | | | | |
|---|---|---|---|---|
| a. Dispersion of C.I. 74160 (pigment blue 15:1) in water/glycerol under the trade name Cosmenyl Blue A4R from Clariant. b. Commercially available under the trade name Plantacare^{®} 1200UP from BASF. | | | | |

### Methods

A polymer/glycerol solution (DAV diluent) was prepared by combining the ingredients in Table 2, and it was used to mimic the viscosity characteristics of saliva.

**Table 2**

| **Ingredient** | **Amount/g** |
|---|---|
| Glycerol | 100 |
| Sodium carboxymethyl cellulose | 10 |
| Formalin | 2 |
| DI Water (Milli Q water, Millipore, USA) | Balance to 2L |

The test sample was mixed with water and DAV diluent at a weight ratio of 1:1:1 to form a toothpaste slurry.

To evaluate tooth whitening efficacy of the samples, the following in vitro test was performed. The WIO index is an index which has been optimized specifically for the evaluation of whiteness in teeth (as described in Journal of Dentistry, volume 36, Supplement 1, 2008, pages 2 to 7). Changes in whiteness was calculated based on L*, a*, b* values which were measured using DigiEye (VeriVide, England).

Twelve bovine blocks were used for each sample. The bovine blocks were brushed for one minute with the toothpaste slurry on a WIRA^{®} brushing machine equipped with toothbrushes. The load of the tooth brushing was 175 g and the automatic brushing operated at a speed of 150 rpm. Thereafter the bovine blocks were rinsed three times each time with 20 mL water by the brushing machine. After this step the L*, a*, b* values of each bovine block were re-measured based on which the changes in WIO values (ΔWIO) from baseline were calculated and statistically analyzed.

### Results

The results are reported in table 3 (error represents standard error for duplicate measurements).

**Table 3**

| | **Samples** | | | |
|---|---|---|---|---|
| | **1** | **2** | **3** | **4** |
| Change in WIO | 5.04±0.54 | 8.44±0.75 | 10.10±2.04 | 7.98±1.45 |

The date indicated that the addition of lauryl glucoside improved the tooth whitening efficacy of samples comprising a copolymer like Pemulen^{™} TR-1 (p<0.01). But for samples comprising a carbomer homopolymer like Carbopol^{®} 971P NF, there was no improvement in tooth whitening efficacy.

### Example 2

This example demonstrated the improvement of tooth whitening efficacy by using a combination of anionic surfactants and alkyl glucosides. All ingredients are expressed by weight percent of the total formulation and as level of active ingredient.

**Table 4**

| **Ingredient** | **Samples** | | | | | | |
|---|---|---|---|---|---|---|---|
| | **1** | **2** | **5** | **6** | **7** | **8** | **9** |
| Water | Balance | Balance | Balance | Balance | Balance | Balance | Balance |
| Sorbitol 70/70 | 45.00 | 45.00 | 45.00 | 45.00 | 45.00 | 45.00 | 45.00 |
| PEG-32 | 2.00 | 2.00 | 2.00 | 2.00 | 2.00 | 2.00 | 2.00 |
| Sodium saccharin | 0.20 | 0.20 | 0.20 | 0.20 | 0.20 | 0.20 | 0.20 |
| Trisodium phosphate | 0.025 | 0.025 | 0.025 | 0.025 | 0.025 | 0.025 | 0.025 |
| Blue Covarine^{a} | 0.025 | 0.025 | 0.025 | 0.025 | 0.025 | 0.025 | 0.025 |
| Pemulen^{™} TR-1 | 0.10 | 0.10 | 0.10 | 0.10 | 0.10 | 0.10 | 0.10 |
| Thickening Silica | 8.2375 | 8.2375 | 8.2375 | 8.2375 | 8.2375 | 8.2375 | 8.2375 |
| Abrasive Silica | 8.00 | 8.00 | 8.00 | 8.00 | 8.00 | 8.00 | 8.00 |
| Sorbosil AC33 | 2.00 | 2.00 | 2.00 | 2.00 | 2.00 | 2.00 | 2.00 |
| Sodium Lauryl sulfate | 1.70 | 0.85 | 1.545 | 1.275 | 0.425 | 0.08 | -- |
| Lauryl glucoside^{b} | -- | 0.85 | 0.155 | 0.425 | 1.275 | 1.62 | 1.70 |
| Perfume | 1.10 | 1.10 | 1.10 | 1.10 | 1.10 | 1.10 | 1.10 |
| Sodium carboxymethyl cellulose | 0.795 | 0.795 | 0.795 | 0.795 | 0.795 | 0.795 | 0.795 |

### Methods

The same protocol was used to evaluate tooth whitening efficacy of the samples as described in Example 1.

### Results

The results are reported in table 5 (error represents standard error for duplicate measurements).

**Table 5**

| | **Samples** | | | | | | |
|---|---|---|---|---|---|---|---|
| | **1** | **2** | **5** | **6** | **7** | **8** | **9** |
| Change in WIO | 5.04± 0.54 | 8.44± 0.75 | 5.43± 0.61 | 8.45± 1.47 | 8.83± 1.40 | 3.67± 1.09 | 5.37± 0.73 |

Samples 2, 6 and 7 showed significantly higher ΔWIO values compared to other samples (p<0.01).

### Example 3

This example demonstrated the effect of polymers on tooth whitening efficacy. All ingredients are expressed by weight percent of the total formulation and as level of active ingredient.

**Table 6**

| **Ingredient** | **Samples** | |
|---|---|---|
| | **10** | **11** |
| Water | Balance | Balance |
| Sorbitol 70/70 | 45.00 | 45.00 |
| PEG-32 | 2.00 | 2.00 |
| Sodium saccharin | 0.20 | 0.20 |
| Trisodium phosphate | 0.025 | 0.025 |
| Blue Covarine^{a} | 0.025 | 0.025 |
| Gantrez S97 | 0.10 | 0.10 |
| Thickening Silica | 8.2375 | 8.2375 |
| Abrasive Silica | 8.00 | 8.00 |
| Sorbosil AC33 | 2.00 | 2.00 |
| Sodium Lauryl sulfate | 1.70 | 0.85 |
| Lauryl glucoside^{b} | -- | 0.85 |
| Perfume | 1.10 | 1.10 |
| Sodium carboxymethyl cellulose | 0.795 | 0.795 |

### Methods

The same protocol was used to evaluate tooth whitening efficacy of the samples as described in Example 1.

### Results

The results are reported in table 7 (error represents standard error for duplicate measurements).

**Table 7**

| | **Samples** | |
|---|---|---|
| | **10** | **11** |
| Change in WIO | 5.01±0.41 | 6.36±1.07 |

For a sample comprising copolymers of methyl vinyl ether and maleic anhydride (Gantrez S97), the addition of lauryl glucoside did not improve the tooth whitening efficacy. Sample 11 showed comparable tooth whitening efficacy (p>0.05) to sample 10.

## Claims

1. An oral care composition comprising:
(a) a copolymer;
(b) an anionic surfactant;
(c) an alkyl glucoside; and
(d) a pigment having a hue angle, h, in the CIELAB system of from 220 to 320 degrees;
wherein the copolymer comprises:
(i) a carboxylic monomer A represented by the formula (1):
CH₂=C(R¹)-COOH (1)
wherein R¹ is a substituent selected from the group consisting of hydrogen, halogen, hydroxyl, lactone, lactam, the cyanogen groups, monovalent alkyl radical, monovalent alkaryl radicals and monovalent cycloaliphatic radicals;
(ii) an acrylic ester monomer B represented by the formula (2):
CH₂=C(R²)-CO(O)-R³ (2)
wherein R² is hydrogen, methyl group or ethyl group, R³ is an alkyl group having from 10 to 30 carbon atoms, preferably from 12 to 22 carbon atoms; and
wherein the anionic surfactant and the alkyl glucoside are present in a weight ratio ranging from 1:8 to 8:1.

2. The oral care composition according to claim 1, wherein the monomer A is acrylic acid, methacrylic acid or ethacrylic acid.

3. The oral care composition according to claim 1 or claim 2, wherein the monomer B is decyl acrylate, lauryl acrylate, stearyl acrylate, behenyl acrylate, myristyl acrylate or the corresponding methacrylates.

4. The oral care composition according to any of the preceding claims, wherein the copolymer is an acrylate/Cw-Cso alkyl acrylate crosspolymer.

5. The oral care composition according to any of the preceding claims, wherein the composition comprises from 0.01 to 10% by weight of the copolymer, preferably from 0.02 to 5%.

6. The oral care composition according to any of the preceding claims, wherein the anionic surfactant is sodium, potassium, ammonium, substituted ammonium salts of alkyl sulfates having 8 to 18 carbon atoms, more preferably 12 to 18 carbon atoms.

7. The oral care composition according to claim 6, wherein the anionic surfactant is sodium lauryl sulfate.

8. The oral care composition according to any of the preceding claims, wherein the composition comprises from 0.01 to 15% by weight of the anionic surfactant, preferably from 0.1 to 10%.

9. The oral care composition according to any of the preceding claims, wherein the alkyl glucoside has a linear or branched alkyl group comprising from 6 to 22 carbon atoms, preferably from 8 to 14, more preferably from 8 to 12.

10. The oral care composition according to claim 9, wherein the alkyl glucoside comprises hexyl glucoside, octyl glucoside, decyl glucoside, lauryl glucoside, isodecyl glucoside, coco glucoside and the corresponding oligosaccharides, and mixtures thereof, preferably decyl glucoside, lauryl glucoside, coco glucoside and mixtures thereof, more preferably lauryl glucoside.

11. The oral care composition according to any of the preceding claims, wherein the anionic surfactant and the alkyl glucoside are present in a weight ratio ranging from 1:5 to 5:1, preferably from 1:3 to 3:1.

12. The oral care composition according to any of the preceding claims, wherein the pigment has a hue angle, h, in the CIELAB system of from 250 to 290.

13. The oral care composition according to claim 12, wherein the pigment is a blue pigment, preferably a phthalocyanine blue pigment.

14. The oral care composition according to claim 13, wherein the phthalocyanine blue pigment is selected from alpha copper phthalocyanines Pigment Blue 15, Pigment Blue 15:1, Pigment Blue 15:2 and mixtures thereof, preferably Pigment Blue 15:1.

15. A non-therapeutic method of whitening teeth of an individual comprising the steps of applying the composition according to any of the preceding claims to at least one surface of the teeth of an individual.

## Patentansprüche

1. Mundpflegezusammensetzung, umfassend:
(a) ein Copolymer;
(b) ein anionisches Tensid;
(c) ein Alkylglucosid; und
(d) ein Pigment mit einem Farbtonwinkel h im CIELAB-System von 220 bis 320 Grad;
wobei das Copolymer umfasst:
(i) ein Carbonsäuremonomer A, dargestellt durch die Formel (1):
CH₂=C(R¹)=COOH (1),
wobei R¹ ein Substituent ist, ausgewählt aus der Gruppe, bestehend aus Wasserstoff, Halogen, Hydroxyl, Lacton, Lactam, Cyanogen-Gruppen, einwertigem Alkylrest, einwertigen Alkaryl-Resten und einwertigen cycloaliphatischen Resten;
(ii) ein Acrylestermonomer B, dargestellt durch die Formel (2):
CH₂=C(R²)-CO(O)-R³ (2),
wobei R² Wasserstoff, eine Methylgruppe oder eine Ethylgruppe ist, R³ eine Alkylgruppe mit 10 bis 30 Kohlenstoffatomen ist, vorzugsweise mit 12 bis 22 Kohlenstoffatomen, und
wobei das anionische Tensid und das Alkylglucosid in einem Gewichtsverhältnis in dem Bereich von 1:8 bis 8:1 vorliegen.

2. Mundpflegezusammensetzung nach Anspruch 1, wobei das Monomer A Acrylsäure, Methacrylsäure oder Ethacrylsäure ist.

3. Mundpflegezusammensetzung nach Anspruch 1 oder Anspruch 2, wobei das Monomer B Decylacrylat, Laurylacrylat, Stearylacrylat, Behenylacrylat, Myristylacrylat oder die korrespondierenden Methacrylate ist.

4. Mundpflegezusammensetzung nach einem der vorhergehenden Ansprüche, wobei das Copolymer ein Acrylat/C₁₀-C₃₀-Alkylacrylat-Crosspolymer ist.

5. Mundpflegezusammensetzung nach einem der vorhergehenden Ansprüche, wobei die Zusammensetzung 0,01 bis 10 Gewichts-% des Copolymers, vorzugsweise 0,02 bis 5%, umfasst.

6. Mundpflegezusammensetzung nach einem der vorhergehenden Ansprüche, wobei das anionische Tensid Natrium-, Kalium-, Ammonium-, substituierte Ammoniumsalze von Alkylsulfaten mit 8 bis 18 Kohlenstoffatomen, bevorzugter mit 12 bis 18 Kohlenstoffatomen, darstellt.

7. Mundpflegezusammensetzung nach Anspruch 6, wobei das anionische Tensid Natriumlaurylsulfat ist.

8. Mundpflegezusammensetzung nach einem der vorhergehenden Ansprüche, wobei die Zusammensetzung 0,01 bis 15 Gewichts-% des anionischen Tensids, vorzugsweise 0,1 bis 10%, umfasst.

9. Mundpflegezusammensetzung nach einem der vorhergehenden Ansprüche, wobei das Alkylglucosid eine lineare oder verzweigte Alkylgruppe aufweist, umfassend 6 bis 22, vorzugsweise 8 bis 14, bevorzugter 8 bis 12 Kohlenstoffatome.

10. Mundpflegezusammensetzung nach Anspruch 9, wobei das Alkylglucosid umfasst Hexylglucosid, Octylglucosid, Decylglucosid, Laurylglucosid, Isodecylglucosid, Kokosglucosid und die entsprechenden Oligosaccharide, und Mischungen davon, bevorzugter Laurylglucosid.

11. Mundpflegezusammensetzung nach einem der vorhergehenden Ansprüche, wobei das anionische Tensid und das Alkylglucosid in einem Gewichtsverhältnis in dem Bereich von 1:5 bis 5:1, vorzugsweise von 1:3 bis 3:1, vorliegt.

12. Mundpflegezusammensetzung nach einem der vorhergehenden Ansprüche, wobei das Pigment einen Farbtonwinkel h im CIELAB-System von 250 bis 290 aufweist.

13. Mundpflegezusammensetzung nach Anspruch 12, wobei das Pigment ein blaues Pigment, vorzugsweise ein Phthalocyaninblaupigment ist.

14. Mundpflegezusammensetzung nach Anspruch 13, wobei das Phthalocyaninblaupigment ausgewählt ist aus den Alpha-Kupferphthalocyaninen Pigment Blue 15, Pigment Blue 15:1, Pigment Blue 15:2 und Mischungen davon, vorzugsweise Pigment Blue 15:1.

15. Nicht-therapeutisches Verfahren zum Aufhellen der Zähne eines Individuums, umfassend die Schritte des Auftragens der Zusammensetzung nach einem der vorhergehenden Ansprüche auf mindestens eine Oberfläche der Zähne eines Individuums.

## Revendications

1. Composition pour le soin oral comprenant :
(a) un copolymère ;
(b) un tensioactif anionique ;
(c) un alkyl glucoside ; et
(d) un pigment ayant un angle de teinte, h, dans le système CIELAB de 220 à 320 degrés ;
dans laquelle le copolymère comprend :
(i) un monomère carboxylique A représenté par la formule (1) :
CH₂=C(R¹)-COOH (1)
dans laquelle R¹ est un substituant choisi dans le groupe consistant en hydrogène, halogène, hydroxyle, lactone, lactame, les groupes cyanogène, radical alkyle monovalent, radicaux alkaryle monovalents et radicaux cycloaliphatiques monovalents ;
(ii) un monomère d'ester acrylique (B) représenté par la formule (2) :
CH₂=C(R²)-CO(O)-R³ (2)
dans laquelle R² est l'hydrogène, groupe méthyle ou groupe éthyle, R³ est un groupe alkyle ayant de 10 à 30 atomes de carbone, de préférence de 12 à 22 atomes de carbone ; et
dans laquelle le tensioactif anionique et l'alkyl glucoside sont présents dans un rapport de masse de 1:8 à 8:1.

2. Composition pour le soin oral selon la revendication 1, dans laquelle le monomère A est l'acide acrylique, l'acide méthacrylique ou l'acide éthacrylique.

3. Composition pour le soin oral selon la revendication 1 ou revendication 2, dans laquelle le monomère B est l'acrylate de décyle, acrylate de lauryle, acrylate de stéaryle, acrylate de béhényle, acrylate de myristyle ou les méthacrylates correspondants.

4. Composition pour le soin oral selon l'une quelconque des revendications précédentes, dans laquelle le copolymère est un copolymère croisé d'acrylate/acrylate d'alkyle en C₁₀-C₃₀.

5. Composition pour le soin oral selon l'une quelconque des revendications précédentes, dans laquelle la composition comprend de 0,01 à 10 % en masse du copolymère, de préférence de 0,02 à 5 %.

6. Composition pour le soin oral selon l'une quelconque des revendications précédentes, dans laquelle le tensioactif anionique est des sels de sodium, potassium, ammonium, ammonium substitué de sulfates d'alkyle ayant de 8 à 18 atomes de carbone, encore mieux de 12 à 18 atomes de carbone.

7. Composition pour le soin oral selon la revendication 6, dans laquelle le tensioactif anionique est le lauryl sulfate de sodium.

8. Composition pour le soin oral selon l'une quelconque des revendications précédentes, dans laquelle la composition comprend de 0,01 à 15 % en masse du tensioactif anionique, de préférence de 0,1 à 10 %.

9. Composition pour le soin oral selon l'une quelconque des revendications précédentes, dans laquelle l'alkyl glucoside présente un groupe alkyle linéaire ou ramifié comprenant de 6 à 22 atomes de carbone, de préférence de 8 à 14, encore mieux de 8 à 12.

10. Composition pour le soin oral selon la revendication 9, dans laquelle l'alkyl glucoside comprend l'hexyl glucoside, octyl glucoside, décyl glucoside, lauryl glucoside, isodécyl glucoside, coco glucoside et les oligosaccharides correspondants, et des mélanges de ceux-ci, de préférence décyl glucoside, lauryl glucoside, coco glucoside et des mélanges de ceux-ci, encore mieux lauryl glucoside.

11. Composition pour le soin oral selon l'une quelconque des revendications précédentes, dans laquelle le tensioactif anionique et l'alkyl glucoside sont présents dans un rapport en masse de 1:5 à 5:1, de préférence de 1:3 à 3:1.

12. Composition pour le soin oral selon l'une quelconque des revendications précédentes, dans laquelle le pigment présente un angle de teinte, h, dans le système CIELAB de 250 à 290.

13. Composition pour le soin oral selon la revendication 12, dans laquelle le pigment est un pigment bleu, de préférence un pigment bleu de phthalocyanine.

14. Composition pour le soin oral selon la revendication 13, dans laquelle le pigment bleu de phthalocyanines est choisi parmi des phtalocyanines de cuivre alpha Pigment Blue 15, Pigment Blue 15:1, Pigment Blue 15:2 et des mélanges de ceux-ci, de préférence Pigment Blue 15:1.

15. Procédé non-thérapeutique de blanchiment des dents d'un individu comprenant les étapes d'application de la composition selon l'une quelconque des revendications précédentes sur au moins une surface des dents d'un individu.
